(19) **Europäisches Patentamt**
**European Patent Office**
**Office européen des brevets**

(11) **EP 4 565 928 B1**

(12) **EUROPÄISCHE PATENTSCHRIFT**

(45) Veröffentlichungstag und Bekanntmachung des Hinweises auf die Patenterteilung:
**17.06.2026  Patentblatt 2026/25**

(21) Anmeldenummer: **23776855.1**

(22) Anmeldetag: **06.09.2023**

(51) Internationale Patentklassifikation (IPC):
*G05B 19/418* (2006.01)    *G05B 19/05* (2006.01)
*G05B 23/02* (2006.01)

(52) Gemeinsame Patentklassifikation (CPC):
**G05B 19/41875; G05B 19/056;** G05B 2219/32077;
G05B 2219/32137

(86) Internationale Anmeldenummer:
**PCT/EP2023/074359**

(87) Internationale Veröffentlichungsnummer:
**WO 2024/056471 (21.03.2024 Gazette 2024/12)**

(54) **ÜBERWACHUNG EXOTHERMER REAKTIONEN IN EINEM REAKTOR**

MONITORING EXOTHERMAL REACTIONS IN A REACTOR

SURVEILLANCE DE RÉACTIONS EXOTHERMIQUES DANS UN RÉACTEUR

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorität:  **14.09.2022  DE 102022209657**

(43) Veröffentlichungstag der Anmeldung:
**11.06.2025  Patentblatt 2025/24**

(73) Patentinhaber: **Siemens Aktiengesellschaft**
**80333 München (DE)**

(72) Erfinder:
• **BIERNATH, Johannes**
**76227 Karlsruhe (DE)**
• **HIRSCH, Volker**
**76889 Steinfeld (DE)**
• **SCHMIDT, Jürgen**
**67434 Neustadt an der Weinstraße (DE)**

(74) Vertreter: **Siemens Patent Attorneys**
**Postfach 22 16 34**
**80506 München (DE)**

(56) Entgegenhaltungen:
EP-B1- 3 621 726          WO-A1-2021/076093
DE-A1- 102018 216 456    US-A1- 2005 246 067
US-A1- 2018 364 747

**EP 4 565 928 B1**

## Beschreibung

Die Erfindung betrifft ein Projektierungssystem und ein Verfahren zur Erstellung eines Programmes zur Überwachung exothermer Reaktionen in einem Reaktor. Die Erfindung betrifft ferner ein Computerprogramm zur Überwachung exothermer Reaktionen in einem Reaktor,

Thermisches Durchgehen ist der Verlust der Temperaturkontrolle einer chemischen Reaktion aufgrund einer exothermen Reaktion, die zu einem explosionshaften Temperaturanstieg im Reaktor führen kann. Der Verlust der Temperaturkontrolle tritt dann ein, wenn die erzeugte Wärme, wie bspw. Reaktionswärme, nicht mehr ausreichend abgeführt werden kann. Die Folgen davon sind eine Erhöhung der Reaktionsgeschwindigkeit und ein daher sich selbstbeschleunigter Prozess, der weitere Energie aus dem Reaktions- oder Zersetzungsprozess freisetzt.

Dadurch steigt die Temperatur im Reaktor und damit in der Regel auch der Druck, Dies wird auch als Durchgehreaktion (engl. "runaway reaction") bezeichnet. Diese Durchgehreaktion bzw. die Selbstbeschleunigung führt zum Ansprechen von Sicherheitseinrichtungen, beispielsweise Sicherheitsventile oder Berstscheiben, und verursacht einen Produktaustritt (oder im schlimmsten Fall eine Explosion des Reaktors), bei dessen plötzlicher Freisetzung hohen Gasemissionen auftreten können, die möglicherwiese sogar entflammbar oder giftig sind.

Im besonderen Maße tritt dieses Problem bei Reaktoren mit exothermen Reaktionen auf, die in einer Semibatch-Fahrweise betrieben werden. Hierbei kann es u.a. zum Einschlafen der Reaktion kommen und zur gleichzeitigen Anreicherung eines zudosierten Eduktes, der sogenannten Akkumulation. Diese Akkumulation kann bei Wiedereinsetzen der Reaktion zu einer exponentiellen Energiefreisetzung führen, die aufgrund der selbstbeschleunigten Reaktion nur schwer zu kontrollieren ist.

Es ist deshalb wünschenswert, das Gefahrenpotential einer Durchgehreaktion schon im Vorfeld über Früherkennungsmethoden zu ermitteln.

Die Publikation Biernath, Johannes, et al. "Model-based zero emission safety concept for reactors with exothermal reactions for chemical plants." Journal of Loss Prevention in the Process Industries 72 (2021); 104494 gibt einen Überblick über bereits bekannte "online"-modellbasierte Früherkennungsmethoden. Diese Methoden nutzen entweder ein Divergenz-Kriterium, ein adiabatisches Kriterium oder ein Akkumulations-Kriterium.

Die Divergenzmethode auf Basis des Divergenz-Kriteriums, die auch Gegenstand der EP 0 882 499 A1 ist, beschreibt, wie über die Temperaturänderungen des Reaktors, die Temperaturänderungsgeschwindigkeit und die Temperaturdifferenzgeschwindigkeit zwischen Reaktor und Kühlmantel ein Volumen aufgespannt wird. Kommt es hierbei zu einer Vergrößerung des Volumens über die Zeit liegt es nahe, dass eine Durchgehreaktion vorliegt. Diese Methode zeigt in den Veröffentlichungen, dass die Zeit zwischen der Erkennung und Eintreten einer Durchgehreaktion nur eine kurze Zeitspanne beträgt, so dass je nach Reaktionsbeschleunigung nur bedingt Gegenmaßnahmen möglich sind.

Bei dem Akkumulations-Kriterium wird eine Akkumulation einer Reaktions-Zugabekomponente (d.h. eines Eduktes) ermittelt und daraus eine maximale Temperatur und ein maximaler Druck in dem Reaktor im Falle einer Durchgehreaktion ermittelt. Zur Ermittlung der Akkumulation werden dabei direkte Konzentrationsmessungen mit Hilfe optischer Methoden oder IR-Spektroskopie oder indirekte Methoden, wie z.B. ein Energie-Bilanz-Ansatz, genannt. Die maximale Temperatur wird beispielsweise mit einer Auslegungstemperatur des Reaktors verglichen und bei Überschreiten eines Schwellwertes wird beispielsweise die Zugabe der Reaktionskomponente unterbunden.

Weitere Details zu dem Akkumulations-Kriterium und zur Berechnung einer adiabatischen Temperaturerhöhung im Fall einer Durchgehreaktion sind in der Publikation Schmidt C., Biernath J., Schmidt J., Denecke J., 2022, Protection of chemical reactors against exothermal runaway reactions with smart overpressure protection devices, Chemical Engineering Transactions, 90, 493-498 DOI:10.3303/CET2290083 beschrieben. Auch hier sind noch weiter Details zu dem Energie-Bilanz-Ansatz offenbart.

Zum weiteren Stand der Technik wird beispielsweise auf die WO 03/103826 A1 (u.a. zum Energie-Bilanz-Ansatz) und auf die WO 00/47632 A1 verwiesen.

Die EP 3 621 726 B1 offenbart ein modellbasiertes Verfahren zur Überwachung der exothermen Reaktionen in einem SemiBatch-Reaktor mit Hilfe des Energie-Bilanz-Ansatzes. Dabei wird eine Akkumulation mindestens einer Reaktionskomponente ermittelt und basierend darauf eine maximale Temperatur in dem Reaktor im Falle einer Durchgehreaktion ermittelt. Dies erfolgt mit Hilfe von Messwerten und ermittelten Stoffdaten von Komponenten in dem Reaktor.

Eine Herausforderung bei den aus dem Stand der Technik bekannten Früherkennungsmethoden liegt dabei in der Überführung in eine sicherheitsgerichtete, speicherprogrammierbare Steuerung (sSPS). Diese Steuerungen limitieren die Anzahl an mathematischen Bausteinen, an zyklische Bearbeitungen, stellen nur eine limitierte Programmiersprache (LVL) bereit und unterliegen zudem normativen Anforderungen u.a. nach IEC 61511.

Eine Programmierung verschiedener Steuer- und Überwachungsprogramme durch eine Verknüpfung von in einer Bibliothek enthaltenen Funktionsbausteinen ist beispielsweise aus der WO 2021/076093 A1 und der DE 10 2018 216456 A1 bekannt.

Es ist deshalb Aufgabe vorliegender Erfindung, ein Projektierungssystem und ein Verfahren zur Erstellung eines Programmes zur Überwachung exothermer Reaktionen in einem Reaktor anzugeben, welches ermöglicht, dass die Überwachung in einer sicherheitsgerichteten, speicherprogrammierbaren Steuerung implementiert werden kann.

Die Lösung dieser Aufgabe gelingt durch ein Projektierungssystem gemäß Patentanspruch 1 und ein Verfahren gemäß Patentanspruch 4. Ein Computerprogramm zur Überwachung exothermer Reaktionen in einem Reaktor ist Gegenstand des Patentanspruchs 7. Vorteilhafte Ausgestaltungen sind jeweils Gegenstand der Unteransprüche.

Ein erfindungsgemäßes Projektierungssystem zur Erstellung eines Programmes zur Überwachung exothermer Reaktionen in einem Reaktor stellt zur Erstellung des Programmes

- zumindest ein erstes Funktionsmodul mit einem mathematischen Modell zur Ermittlung einer maximalen Temperatur und/oder einem maximalen Druck in dem Reaktor im Falle einer Durchgehreaktion anhand von Messwerten und anhand von Stoffdaten von Komponenten in dem Reaktor, vorzugsweise über eine Ermittlung von Konzentrationen von Komponenten in dem Reaktor, und
- zumindest ein zweites Funktionsmodul zur Ermittlung der Stoffdaten, insbesondere einer Wärmekapazität, einer Dichte, eines Dampfdruckes, einer Leitfähigkeit, einer Löslichkeit und/oder einer Viskosität einer oder mehrerer Komponenten in dem Reaktor,

bereit.

Bei einem erfindungsgemäßen Verfahren zur Erstellung eines Programmes zur Überwachung exothermer Reaktionen in einem Reaktor wird das Programm aus

- zumindest einem ersten Funktionsmodul mit einem mathematischen Modell zur Ermittlung einer maximalen Temperatur und/oder einem maximalen Druck in dem Reaktor im Falle einer Durchgehreaktion anhand von Messwerten und anhand von Stoffdaten von Komponenten in dem Reaktor, vorzugsweise über eine Ermittlung von Konzentrationen von Komponenten in dem Reaktor, und
- zumindest einem zweiten Funktionsmodul zur Ermittlung der Stoffdaten, insbesondere einer Wärmekapazität, einer Dichte, eines Dampfdruckes, einer Leitfähigkeit, einer Löslichkeit und/oder einer Viskosität einer oder mehrerer Komponenten in dem Reaktor,

erstellt.

Ein erfindungsgemäßes Computerprogramm zur Überwachung exothermer Reaktionen in einem Reaktor umfasst

- zumindest ein erstes Funktionsmodul mit einem mathematischen Modell zur Ermittlung einer maximalen Temperatur und/oder einem maximalen Druck in dem Reaktor im Falle einer Durchgehreaktion anhand von Messwerten und anhand von Stoffdaten von Komponenten in dem Reaktor, vorzugsweise über eine Ermittlung von Konzentrationen von Komponenten in dem Reaktor, und
- zumindest ein zweites Funktionsmodul zur Ermittlung der Stoffdaten, insbesondere einer Wärmekapazität, einer Dichte, eines Dampfdruckes, einer Leitfähigkeit, einer Löslichkeit und/oder einer Viskosität einer oder mehrerer Komponenten in dem Reaktor.

Durch einen derartigen modularen Aufbau, eine flexible Verschaltung derartiger Module und deren Parametrierung ist eine leichte Anpassung an unterschiedliche Prozesse, Stoffe und Anlagengrößen möglich. Die Erstellung des Programmes kann somit sehr effizient erfolgen und systematische Fehler können vermieden werden.

Bei dem mathematischen Modell erfolgt die Ermittlung der maximalen Temperatur und/oder des maximalen Drucks in dem Reaktor im Falle einer Durchgehreaktion anhand von Messwerten und anhand von Stoffdaten von Komponenten in dem Reaktor über eine Ermittlung von Konzentrationen von Komponenten in dem Reaktor. Mit anderen Worten ist die Ermittlung der Konzentrationen ebenfalls von dem mathematischen Modell umfasst und es erfolgt zuerst eine Ermittlung von Konzentrationen von Komponenten in dem Reaktor und aufbauend darauf erfolgt dann die Ermittlung der maximalen Temperatur und/oder des maximalen Drucks in dem Reaktor.

Das zweite Funktionsmodul kann dabei auch Reinstoffgleichungen beinhalten, es kann aber beispielsweise auch nur für eine Einheitenumrechnung genutzt werden.

Das erstes Funktionsmodul und/oder das zweite Funktionsmodul kann dabei auch Anlagengrößen als Eingangsgrößen haben (z.B. ein Reaktorvolumen).

Für eine einfache Zuspielung und Ermittlung von Stoffdaten umfasst zumindest das zweite Funktionsmodul dabei eine Schnittstelle zur Erfassung von Konstanten von Reinstoffgleichungen, insbesondere aus einer Stoffdatenbank.

Für eine einfache Programmierung sind die Funktionsmodule vorzugsweise als Elemente (z.B. als Bausteine oder sogenanntes "Modultypicals") in einer Bibliothek bereitgestellt.

Gemäß der Erfindung umfasst das Programm Befehle, die bei der Ausführung des Programmes durch einen Computer diesen veranlassen, ein Verfahren auszuführen, bei dem zur Überwachung der exothermen Reaktionen in dem Reaktor eine Akkumulation mindestens einer Reaktionskomponente in dem Reaktor ermittelt und basierend darauf eine maximale Temperatur und/oder ein maximaler Druck in dem Reaktor im Falle einer Durchgehreaktion ermittelt wird. Die Akkumulation der Reaktionskomponente kann dabei mit Hilfe von Messwerten, wie z.B. Temperatur, Druck und/oder Schallgeschwindigkeit in dem Reaktor ermittelt werden. Vorzugsweise erfolgt die Überwachung dabei "online" (d.h. in Echtzeit) auf Basis von "online"-Messwerten.

Wie sich herausgestellt hat, kann die Akkumulation der Reaktionskomponente und basierend darauf eine maximale Temperatur und/oder ein maximaler Druck dann besonders einfach, und somit auch für eine Implementierung in einer sicherheitsgerichteten, speicherprogrammierbaren Steuerung geeignet, ermittelt werden, wenn die Akkumulation der Reaktionskomponente mit Hilfe von Messwerten einer Schallgeschwindigkeit in dem Reaktor ermittelt wird. Dieses Verfahren kann sehr einfach in verschiedene aufeinander aufbauende Teile untergliedert werden, die dann als jeweils separate Funktionsmodule implementiert werden können. Als Eingangsgrößen sind beispielsweise nur die Messwerte, Stoffdaten und kinetische Daten der Produktion notwendig. Die Stoffdaten können beispielsweise (automatisch oder über einen Operator) von einer Stoffdatenbank wie dem VDI Wärmeatlas erfasst werden. Für die Ermittlung der maximalen Temperatur und/oder des maximalen Drucks sind dann nur relativ einfache mathematische Berechnungen notwendig, die auch in einer sicherheitsgerichteten, speicherprogrammierbaren Steuerung mit ihren begrenzten mathematischen Möglichkeiten und der zyklischen Programmbearbeitung implementiert werden können.

Eine Schallgeschwindigkeitsmessung zur Ermittlung von Gasanteilen in einem Gasgemisch ist grundsätzlich bereits aus der Publikation Bates R., et al "Implementation of Ultrasonic Sensing for High Resolution Measurement of Binary Gas Mixture Fractions", Sensors 2014, 14, 11260-11276; doi:10.3390/s240611260 bekannt. Wie sich herausgestellt hat, kann eine derartige Messung der Schallgeschwindigkeit von großem Vorteil auch für eine Messung der Stoffmengenkonzentrationen in einem Reaktor genutzt werden und darauf aufbauend mit relativ einfachen Gleichungen beispielsweise eine Konzentration der Reaktionskomponente und/oder anderer Komponenten in dem Reaktor und deren Akkumulation ermittelt werden.

Grundsätzlich ist dabei eine Messung in einer Flüssigkeit oder in einem Gasraum des Reaktors möglich.

Vorzugsweise bezieht sich die Schallgeschwindigkeit auf eine Geschwindigkeit eines Schalls im Ultraschallbereich, d.h. Schallwellen oberhalb der menschlichen Hörgrenze. Wie sich herausgestellt hat, ist dieser Schallbereich gut messbar und hat wenig Störeinflüsse.

Gemäß einer vorteilhaften Ausgestaltung erfolgt die Ermittlung der maximalen Temperatur und/oder des maximalen Drucks zusätzlich mit Hilfe von Messwerten einer Dichte, einer Temperatur und einem Druck in dem Reaktor (oder von Größen, aus denen die vorgenannten Größen ableitbar sind). Hierdurch können die mathematischen Berechnungen der Akkumulation der Reaktionskomponente und somit die Ermittlung der maximalen Temperatur und/oder des maximalen Drucks noch weiter vereinfacht werden.

Gemäß einer weiteren vorteilhaften Ausgestaltung wird mit Hilfe der Messwerte eine Konzentration von Komponenten in dem Reaktor ermittelt.

Gemäß einer weiteren vorteilhaften Ausgestaltung wird ein Ausgangssignal für eine Aktivierung einer Sicherheitsreaktion erzeugt, wenn die ermittelte maximale Temperatur und/oder der maximale Druck einen Schwellwert überschreitet. Das Ausgangssignal kann beispielsweise einen Alarm für einen Operator des Reaktors auslösen, der dann seinerseits eine Sicherheitsreaktion auslöst, oder aber direkt eine automatische Sicherheitsreaktion auslösen.

Der Schwellwert ist vorzugsweise aus einem Auslegungsgrenzwert des Reaktors oder einem Ansprechwert einer Sicherheitseinrichtung des Reaktors abgeleitet.

Die Sicherheitsreaktion umfasst im einfachsten Fall eine Rediazierurig oder eine Beendigung (Stop) einer Zufuhr der Reaktionskomponente zu dem Reaktor und/oder eine Erhöhung einer Kühlung des Reaktors.

Da die Gefahr von Durchgehreaktionen bei einer Semi-Batch-Fahrweise besonders groß ist, kommt es mit großem Vorteil dann zum Einsatz, wenn der Reaktor in einer Semi-Batch-Fahrweise betrieben wird.

Gemäß einer besonders vorteilhaften Ausgestaltung ist das Programm ein fehlersicheres Programm einer sicherheitsgerichteten, insbesondere speicherprogrammierbaren, Steuerung.

Das vorstehend beschriebene Projektierungssystem, Projektierungsverfahren und Computerprogramm können auch in Fällen eingesetzt werden, in denen eine Akkumulation einer Reaktionskomponente auch z.B. ohne Messwerte einer Schallgeschwindigkeit ermittelt wird, beispielsweise mit Hilfe optischer Verfahren oder einer Energie-Bilanz-Berechnung. Sie sind besonders zur Erstellung von fehlersicheren Programmen, insbesondere für eine sicherheitsgerichtete, speicherprogrammierbare Steuerung geeignet.

Die Erfindung sowie weitere vorteilhafte Ausgestaltungen der Erfindung gemäß Merkmalen der Unteransprüche werden im Folgenden anhand von Ausführungsbeispielen in den Figuren näher erläutert, aber beschränken sich nicht auf diese; darin zeigen:

FIG 1    in schematischer Darstellung einen Reaktor mit einer Steuerung zur Überwachung einer exothermen Reaktion in dem Reaktor,

FIG 2    ein Beispiel für eine Erstellung eines Programmes zur Überwachung einer exothermen Reaktion in dem Reaktor.

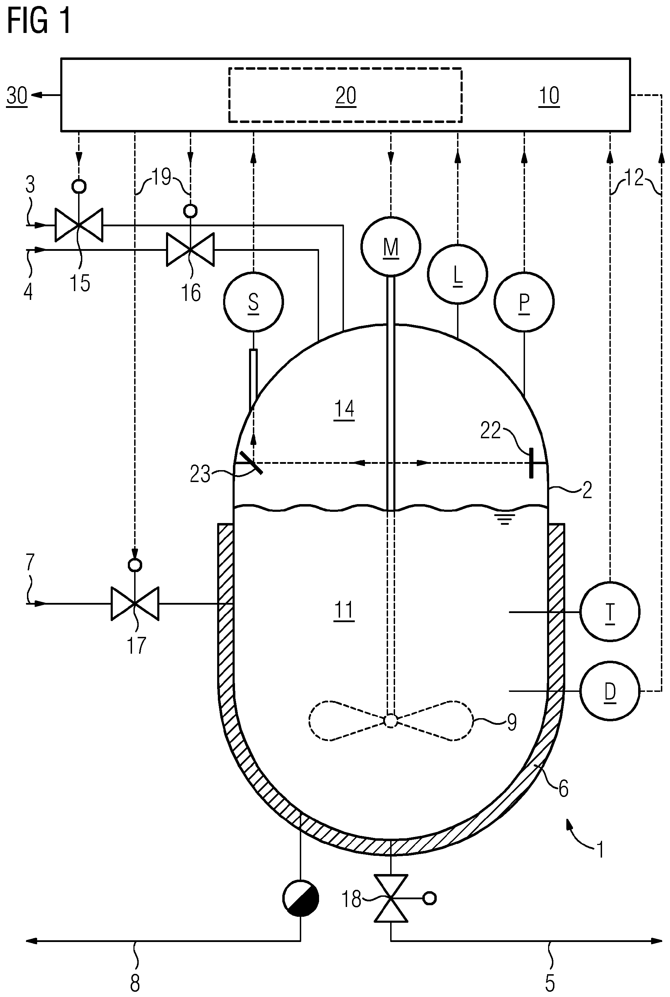

Die FIG 1 zeigt in vereinfachter schematischer Darstellung einen chemischen Reaktor 1 mit einem Reaktorkessel 2, mehreren Zuläufen zu dem Kessel 2 für Reaktanten und weitere Hilfsstoffe (beispielhaft sind in FIG 1 zwei Zuläufe 3, 4 dargestellt), einem Ablauf 5 aus dem Kessel 2 für ein Reaktionsprodukt, und einen Kühlmantel 6, dem über eine Zulauf 7 ein Kühlmittel zuführbar und über einen Ablauf 8 abführbar ist. In dem Kessel 2 ist ein Rührer 9 angeordnet, dessen Welle nach oben aus dem Kessel herausgeführt ist und von einem Motor M angetrieben wird. In dem Kessel 2 befindet sich unten ein flüssiges Phasengemisch 11 und oben ein Gasraum 14.

Mit Hilfe verschiedener Messwertaufnehmer (Sensoren) können physikalische Größen im Reaktor und in den Zu- und Abläufen gemessen werden. Beispielhaft sind in FIG 1 ein Drucksensor P zur Messung des Drucks im Reaktorkessel 2, ein Temperatursensor T zur Messung der Temperatur des flüssigen Phasengemisches 11 im Reaktorkessel 2, ein Dichtemesser D zur Messung einer mittleren Dichte des flüssigen Phasengemisches 11, ein Füllstandmessgerät L zur Messung eines Füllstands des flüssigen Phasengemisches 11 und ein Schallgeschwindigkeitsmessgerät S zur Messung einer Schallgeschwindigkeit in dem Gasraum 14. Es könne noch weitere Messwertaufnehmer vorhanden sein, wie z.B. Durchflussmesser in den Zuläufen und Abläufen, Temperatursensoren im Kühlmantel 6, etc.

Eine Steuerung 10 dient zur Steuerung und Überwachung des Reaktors 1. Vorzugsweise handelt es sich um eine sicherheitsgerichtete, fehlersichere Steuerung wie beispielsweise eine fehlersichere SIMATIC S7 der Anmelderin. Die Steuerung erfasst die von den Messwertaufnehmern (Sensoren) erzeugten Messwerte und ist hierzu über schematisch dargestellte Signalleittingen 12 mit den Messwertaufnehmern P, T, D, L, S verbunden. Die Steuerung 10 kann wiederum über Aktoren wie z.B. Ventile in den Zu- und Abläufen (siehe Ventile 15, 16, 17, 18) eine Zufuhr bzw. Abfuhr von Reaktanten, Hilfsstoffen, Reaktionsprodukten und Kühlmittel steuern und ist hierzu über Steuerleitungen 19 mit den Aktoren verbunden.

In dem Reaktor 1 findet eine exotherme Reaktion von Reaktanten statt. Der Reaktor wird dabei beispielsweise in einer Semi-Batch-Fahrweise betrieben. Durch eine fehlersichere Programmierung 20 in der Steuerung 10 wird nun ein modellbasiertes kontinuierliches online-Verfahren zur Überwachung der exothermen Reaktion in dem Reaktor durchgeführt, d.h. die Überwachung erfolgt in Echtzeit.

Bei diesem Verfahren wird eine Akkumulation mindestens einer Reaktionskomponente (üblicherweise eines Eduktes) in dem Reaktor ermittelt und basierend darauf eine maximale Temperatur und/oder ein maximaler Druck in dem Reaktor im Falle einer Durchgehreaktion ermittelt. Die Akkumulation der Reaktionskomponente wird dabei von der fehlersichere Programmierung 20 mit Hilfe der vorstehend beschriebenen Messwerte der Schallgeschwindigkeit, der Temperatur, der Dichte und des Drucks in dem Reaktor 1 ermittelt.

Die Messung der Schallgeschwindigkeit durch das Schallgeschwindigkeitsmessgerät S erfolgt vorzugsweise im Ultraschallbereich. Für das Schallgeschwindigkeitsmessgerät S kann beispielhaft ein Ultraschallsensor vom Typ Echomax xps-10 in Verbindung mit einem SITRANS LUT400 Ultraschall-Auswertegerät der Anmelderin zum Einsatz kommen. Das Schallgeschwindigkeitsmessgerät S bzw. die zugehörigen Schallsensoren sitzen beispielsweise oben auf dem Kessel 2 und speisen somit von oben den Schall in vertikaler Richtung in den Kessel bzw. empfangen oben einen von einem Reflektorelement 22 reflektierten Schall. Das Reflektorelement 22 ist im Gasraum 14 im Kessel 2 angeordnet. Grundsätzlich ist aber auch ein Einspeisen bzw. Empfangen des Schalls in horizontaler Richtung möglich

Zur Verlängerung der Wegstrecke und somit zur Erhöhung der Genauigkeit der Messung wird der Schall über ein Umlenkelement 23, das ebenfalls im Gasraum 14 im Kessel 2 angeordnet ist, in eine Richtung senkrecht zur Einspeiserichtung (d.h. in eine horizontale Richtung) umgelenkt. Der somit umgelenkte Schall wird dann von dem Reflektorelement 22 reflektiert und über den gleichen Weg - nur im umgekehrter Richtung (d.h. wiederum über das Umlenkelement 23) - zu dem Schallgeschwindigkeitsmessgerät S zurückgeführt.

In der Steuerung 10 ist ein Schwellwert für eine maximale Temperatur und/oder einen maximalen Druck abgespeichert. Der Schwellwert ist beispielsweise aus einem Auslegungsgrenzwert des Reaktors oder einem Ansprechwert einer Sicherheitseinrichtung des Reaktors abgeleitet. Die Steuerung 10 bzw. das fehlersichere Programm 20 vergleicht nun kontinuierlich die ermittelte maximale Temperatur und/oder den maximalen Druck und erzeugt bei einem Überschreiten entweder ein Ausgangssignal 30 (z.B. einen Alarm) für einen Operator, der dann eine Sicherheitsreaktion auslösen kann, oder löst automatisch selbst eine Sicherheitsreaktion aus. Eine mögliche Sicherheitsreaktion könnte beispielsweise eine Erhöhung der Kühlmittelzufuhr zu dem Kühlmantel oder eine Reduzierung oder Beendigung einer Zufuhr des Edukts zu dem Reaktor 1 sein.

Im Folgenden soll nun das Ausführungsbeispiel für eine beispielhafte, aber nicht nur darauf zu beschränkende, chemische Reaktion erläutert werden, Hierbei handelt es sich um eine Veresterung von Essigsäureanhydrid mit Methanol zur Erzeugung Essigsäure und Essigsäuremethylesther (d.h. 4 Kornponenten):

$$(CH3CO)_2O_{(l)} + CH_3OH_{(l)} \rightarrow CH_3COOCH_{3(l)} + CH_3COOH_{(l)}$$

Abkürzungen:

A = Essigsäureanhydrid
B = Methanol
C = Essigsäuremethylesther (Nebenprodukt)
D = Essigsäure (Hauptprodukt)

1) Messung der mittleren Flüssigkeitsdichte und Mischung über den reziproken Ansatz der mit dem Massenanteil gewichteten Reinstoffdichten (z.B. nach VDI WA):

$$\rho_m = \left( \frac{\frac{x_A M_A}{x_A M_A + x_B M_B + x_C M_C + x_D M_D}}{\rho_A} + \frac{\frac{x_B M_B}{x_A M_A + x_B M_B + x_C M_C + x_D M_D}}{\rho_B} \right.$$
$$+ \frac{\frac{x_C M_C}{x_A M_A + x_B M_B + x_C M_C + x_D M_D}}{\rho_C}$$
$$\left. + \frac{\frac{x_D M_D}{x_A M_A + x_B M_B + x_C M_C + x_D M_D}}{\rho_D} \right)^{-1}$$

$\rho_m$: mittlere Dichte der Flüssigphase (gemessen mit Dichtemesser D)
$\rho_i$: Dichte der Komponente i (i = A, B, C, D)
$x_i$: Stoffmengenanteil der Komponente i in der Flüssigphase (i = A, B, C, D)
$M_i$: Molare Masse der Komponente i in der Flüssigphase (i = A, B, C, D)

2) Messung Druck im Gasraum 14 mit dem Drucksensor P (setzt sich aus Stickstoffüberlagerung und Partialdrücke der Komponenten zusammen):

$$p = x_A p_A^s + x_B p_B^s + x_C p_C^s + x_D p_D^s + \frac{n_{N2} \cdot R \cdot T}{V_g}$$

p: Druck im Gasraum 14 (gemessen)
$p^s_i$: partieller Sättigungsdruck der Komponente i (i = A, B, C, D)
$n_{N2}$: Stoffmenge Stickstoff
R: molare Gaskonstante
T: absolute Temperatur
$V_g$: Volumen des Gasraums

3) Messung Schallgeschwindigkeit im Gasraum 14:

$$a_m^2 = \frac{\frac{c_{p,g,N2} + x_B \frac{p_C^s}{p}(c_{p,g,B} - c_{p,g,N2}) + x_C \frac{p_C^s}{p}(c_{p,g,C} - c_{p,g,N2})}{c_{p,v,N2} + x_B \frac{p_C^s}{p}(c_{p,v,B} - c_{p,v,N2}) + x_C \frac{p_C^s}{p}(c_{p,v,C} - c_{p,v,N2})}}{M_{N2} + x_B \frac{p_C^s}{p}(M_B - M_{N2}) + x_C \frac{p_C^s}{p}(M_C - M_{N2})}$$

$c_{p,g,i}$: spezifische Wärmekapazität der Komponente i (i = B, C, N2) bei konstantem Druck
$c_{p,v,i}$: spezifische Wärmekapazität der Komponente i (i = B, C, N2) bei konstantem Volumen
p: Druck im Gasraum 14
$M_i$: Molare Masse der Komponente i (i = B, C, N2)
$p_c{}^s_i$: kritischer Sättigungsdampfdruck der Komponente i

Bemerkung: Bei obiger Formel für die Schallmessung wurden zur Vereinfacharig der Berechnung Komponenten, die vergleichsweise gering dampfbildend sind (hier die Komponenten A und D) nicht berücksichtigt.

4) Schließbedingung der Aufteilung der Komponenten in der Flüssigphase:

$$1 = x_A + x_B + x_C + x_D$$

Es liegen somit 4 unbekannte Stoffmengenanteile (bzw. Konzentrationen) und 4 Gleichungen hierzu vor, d.h. dieses System ist algebraisch lösbar.

Die weiteren Schritte sind:

a) Auflösung des Gleichungssystems nach der akkumulierten Menge $x_B$ der zudosierten Menge B.

b) Bestimmung der akkumulierten Stoffmengenkonzentration $c_B$ der zudosierten Menge B aus der akkumulierten Menge $x_B$ in bekannter Weise über eine Stoffmengenumrechung.

c) Bestimmung der adiabaten Temperaturerhöhung mittels der akkumulierten Masse

$$\Delta T_{ad} = c_B \frac{\Delta H_R}{\overline{\rho}\ \overline{c}_p}$$

$\underline{c_B}$ = akkumulierte Stoffmengenkonzentration der zudosierten Menge B
$\overline{p}$: mittlere Dichte der Flüssigphase
$\overline{c}_p$: mittlere spezifische Wärmekapazität über alle Komponenten der Flüssigphase
$\Delta HR$: Reaktionsenthalpie
$\Delta T_{ad}$: adiabate Temperaturerhöhung

d) Bestimmung der adiabaten Druckerhöhung $\Delta p_{ad}$ aus der adiabate Temperaturerhöhung $\Delta T_{ad}$ mit Hilfe der allgemeinen Gasgleichung.

Ausgehend von der aktuellen Temperatur bzw. dem aktuellen Druck im Reaktor kann anhand der adiabaten Temperaturerhöhung bzw. der adiabaten Druckerhöhung eine maximale Temperatur bzw. ein maximaler Druck im Reaktor im Fall einer Durchgehreaktion ermittelt werden.

In dem Beispiel von FIG 1 wurden 4 Stoffkomponenten verwendet bzw. Gleichungen für 4 Stoffkomponenten aufgestellt. In analoger Weise können natürlich auch weniger oder mehr Stoffkomponenten zum Einsatz kommen bzw. entsprechende Gleichungen für eine größere oder kleinere Zahl von Komponenten aufgestellt werden.

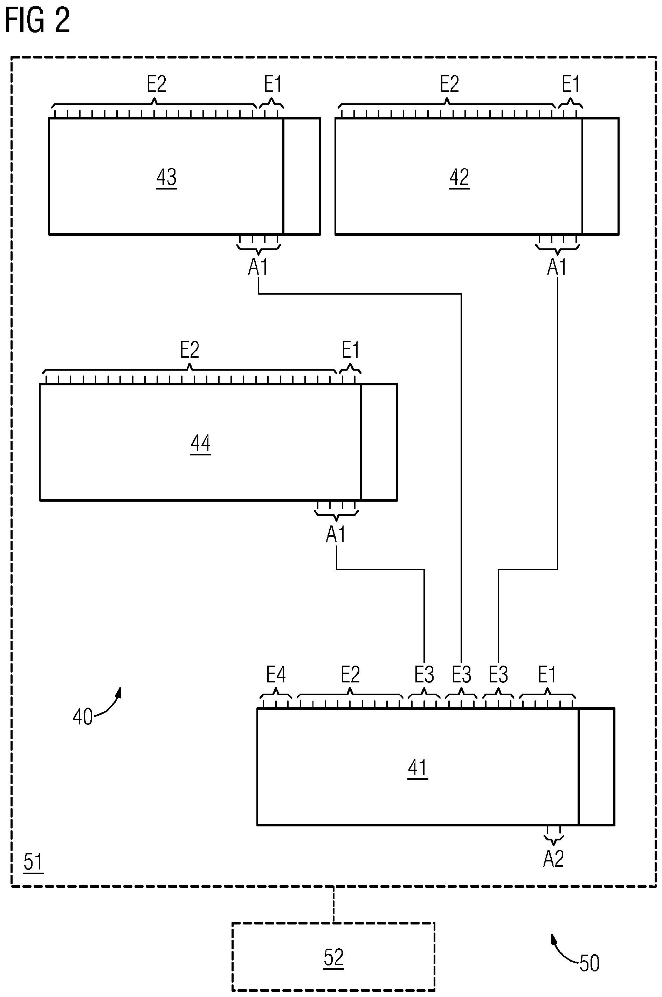

FIG 2 zeigt beispielhaft eine Erstellung eines Programmes zur Überwachung exothermer Reaktionen in einem Reaktor, insbesondere für das fehlersichere Programm 20 in der Steuerung 10 von FIG 1.

FIG 2 zeigt dabei eine Sicht, die einem Projekteur zur Erstellung des Programmes auf einer Bedienoberfläche 51 eines Projektierungssystems 50 angeboten wird. Das Projektierungssystem 50 umfasst weiterhin eine zentrale Recheneinheit 52 wie beispielsweise einen PC.

Die fehlersichere Programmierung umfasst mehrere Funktionsmodule 41, 42, 43, 44.

Ein erstes Funktionsmodul 41 umfasst ein mathematisches Modell, wie das beispielhaft vorstehend beschriebene Modell, zur Ermittlung der maximalen Temperatur und/oder des maximalen Drucks im Fall einer Durchgehreaktion anhand von Messwerten und anhand von Stoffdaten der Komponenten in dem Reaktor. Je nach Bedarf können noch weitere Funktionsmodelle zur Ermittlung der maximalen Temperatur und/oder des maximalen Drucks im Fall einer Durchgehreaktion anhand von Messwerten und anhand von Stoffdaten der Komponenten in dem Reaktor ergänzt werden.

Die Funktionsmodule 42, 43, 44 dienen zur Ermittlung und Bereitstellung der Stoffdaten (und ggf. weiterer kinetischer Daten) einer oder mehrerer Komponenten in dem Reaktor für das Funktionsmodul 41, das Funktionsmodul 42 dient zur Ermittlung von Dampfdrücken, das Funktionsmodul 43 dient zur Ermittlung von Dichten und das Funktionsmodul 44 dient zur Ermittlung von Wärmekapazitäten. Je nach Bedarf können noch weitere Funktionsmodelle zur Ermittlung und Bereitstellung der Stoffdaten (und ggf. weiterer kinetischer Daten) einer oder mehrerer Komponenten in dem Reaktor ergänzt werden.

Für die Ermittlung von Stoffdaten (und ggf. weiterer kinetischer Daten) sind in jedem der Funktionsmodule 41, 42, 43, 44 Reinstoffgleichungen hinterlegt.

Jedes der Funktionsmodule 41, 42, 43, 44 hat Eingänge E1 für Messwerte und Eingänge E2 für Konstanten der Reinstoffgleichungen. Außerdem können Eingänge E4 für Anlagengrößen (z.B. ein Reaktorvolumen) vorhanden sein.

Die Konstanten der Reinstoffgleichungen können beispielsweise von einem Operator oder automatisiert aus einer Reinstoffdatenbank wie z.B. dem VDI Wärmeatlas erfasst werden.

Jedes der Funktionsmodule 42, 43, 44 hat Ausgänge A1 für die ermittelten Stoffdaten, die wiederum mit entsprechenden Eingängen E3 des Funktionsmoduls 41 verbunden sind. Das Funktionsmodul 41 hat Ausgänge A2, die beispielsweise bereits eine Information über ein Überschreiten eines Schwellwertes, d.h. eine kommende Gefahrensituation, ausgeben. Es kann aber über die Ausgänge A2 beispielsweise auch nur eine ermittelte maximale Temperatur und/oder maximaler Druck ausgegeben werden und der Vergleich mit einem Schwellwert erfolgt außerhalb des Funktionsbausteins 41.

Die Ausgänge A1, A2 können dabei auch ein BAD-Signal umfassen, das eine fehlerhaften Berechnung signalisiert. Beispielsweise sind die Funktionsmodule 42, 43, 44 über jeweils ein BAD-Signal an das Funktionsmodul 41 angebunden, so dass bei fehlerhaften Berechnungen auch das Funktionsmodul 41 am Ausgang A2 ein BAD-Signal anliegen hat, welches als Alarm an einen Operator ausgegeben werden kann.

Vorzugsweise werden die Funktionsmodule als Elemente (sogenannte Modultypicals) in einer Bausteinbibliothek für die fehlersichere Programmierung der Steuerung 10 bereitgestellt.

Aufgrund des beschriebenen modularen Aufbaus kann ein Projekteur flexibel für seinen jeweiligen Anwendungsfall aus der Bibliothek die benötigten Module auswählen, miteinander verschalten und parametrieren. Die Erstellung des Programmes kann somit sehr effizient und frei von systematischen Fehlern erfolgen.

Das Programm kann, aber muss nicht, in einer sicherheitsgerichteten Steuerung zum Einsatz kommen. Beispielsweise kann das Programm auch als Überwachungsfunktion in einer nicht sicherheitsgerichteten Applikation (z.B. cloudbasiert) zum Einsatz kommen. Diese Applikation kann beispielsweise zur Überwachung einer Produktqualität dienen.

**Patentansprüche**

1. Projektierungssystem zur Erstellung eines Programmes zur Überwachung exothermer Reaktionen in einem Reaktor, wobei das Projektierungssystem zur Erstellung des Programmes

   - zumindest ein erstes Funktionsmodul mit einem mathematischen Modell zur Ermittlung einer maximalen Temperatur und/oder einem maximalen Druck in dem Reaktor im Falle einer Durchgehreaktion anhand von Messwerten von physikalischen Größen im Reaktor und anhand von Stoffdaten von Komponenten in dem Reaktor über eine Ermittlung von Konzentrationen von Komponenten in dem Reaktor, und
   - zumindest ein zweites Funktionsmodul zur Ermittlung der Stoffdaten, insbesondere einer Wärmekapazität, einer Dichte, eines Dampfdruckes, einer Leitfähigkeit, einer Löslichkeit und/oder einer Viskosität einer oder mehrerer Komponenten in dem Reaktor,

   bereitstellt,

   wobei zumindest das zweite Funktionsmodul eine Schnittstelle zur Erfassung von Konstanten von Reinstoffgleichungen, insbesondere aus einer Stoffdatenbank, umfasst, und
   wobei das Programm Befehle umfasst, die bei der Ausführung des Programmes durch einen Computer diesen veranlassen, ein Verfahren auszuführen, bei dem zur Überwachung der exothermen Reaktionen in dem Reaktor eine Akkumulation mindestens einer Reaktionskomponente in dem Reaktor ermittelt wird und basierend darauf eine maximale Temperatur und/oder ein maximaler Druck in dem Reaktor im Falle einer Durchgehreaktion ermitteilt wird.

2. Projektierungssystem nach Anspruch 1, wobei das Programm ein fehlersicheres Programm einer sicherheitsgerichteten, insbesondere speicherprogrammierbaren, Steuerung ist.

3. Projektierungssystem nach einem der Ansprüche 1 bis 2, wobei die Funktionsmodule als Elemente einer Bibliothek für eine fehlersichere Programmierung einer Steuerung bereitgestellt sind.

4. Verfahren zur Erstellung eines Programmes zur Überwachung exothermer Reaktionen in einem Reaktor, wobei das Programm aus

   - zumindest einem ersten Funktionsmodul mit einem mathematischen Modell zur Ermittlung einer maximalen Temperatur und/oder einem maximalen Druck in dem Reaktor im Falle einer Durchgehreaktion anhand von Messwerten von physikalischen Größen im Reaktor und anhand von Stoffdaten von Komponenten in dem Reaktor über eine Ermittlung von Konzentrationen von Komponenten in dem Reaktor, und
- zumindest einem zweiten Funktionsmodul zur Ermittlung der Stoffdaten, insbesondere einer Wärmekapazität, einer Dichte, eines Dampfdruckes, einer Leitfähigkeit, einer Löslichkeit und/oder einer Viskosität einer oder mehrerer Komponenten in dem Reaktor,

erstellt wird,

wobei zumindest das zweite Funktionsmodul eine Schnittstelle zur Erfassung von Konstanten von Reinstoffgleichungen, insbesondere aus einer Stoffdatenbank, umfasst, und
wobei das Programm Befehle umfasst, die bei der Ausführung des Programmes durch einen Computer diesen veranlassen, ein Verfahren auszuführen, bei dem zur Überwachung der exothermen Reaktionen in dem Reaktor eine Akkumulation mindestens einer Reaktionskomponente in dem Reaktor ermittelt wird und basierend darauf eine maximale Temperatur und/oder ein maximaler Druck in dem Reaktor im Falle einer Durchgehreaktion ermittelt wird,

5. Verfahren nach Anspruch 4, wobei das Programm ein fehlersicheres Programm einer sicherheitsgerichteten, insbesondere speicherprogrammierbaren, Steuerung ist.

6. Verfahren nach einem der Ansprüche 4 bis 5, wobei die Funktionsmodule als Elemente einer Bibliothek für eine fehlersichere Programmierung einer Steuerung bereitgestellt sind.

7. Computerprogramm zur Überwachung exothermer Reaktionen in einem Reaktor, umfassend

- zumindest ein erster Funktionsmodul mit einem mathematischen Modell zur Ermittlung einer maximalen Temperatur und/oder einem maximalen Druck in dem Reaktor im Falle einer Durchgehreaktion anhand von Messwerten von physikalischen Größen im Reaktor und anhand von Stoffdaten von Komponenten in dem Reaktor über eine Ermittlung von Konzentrationen von Komponenten in dem Reaktor, und
- zumindest ein zweites Funktionsmodul zur Ermittlung der Stoffdaten, insbesondere einer Wärmekapazität, einer Dichte, eines Dampfdruckes, einer Leitfähigkeit, einer Löslichkeit und/oder einer Viskosität einer oder mehrerer Komponenten in dem Reaktor,
wobei zumindest das zweite Funktionsmodul eine Schnittstelle zur Erfassung von Konstanten von Reinstoffgleichungen, insbesondere aus einer Stoffdatenbank, umfasst, und
wobei das Programm Befehle umfasst, die bei der Ausführung des Programmes durch einen Computer diesen veranlassen, ein Verfahren auszuführen, bei dem zur Überwachung der exothermen Reaktionen in dem Reaktor eine Akkumulation mindestens einer Reaktionskomponente in dem Reaktor ermittelt wird und basierend darauf eine maximale Temperatur und/oder ein maximaler Druck in dem Reaktor im Falle einer Durchgehreaktion ermittelt wird.

8. Computerprogramm nach Anspruch 7, wobei das Programm ein fehlersicheres Programm einer sicherheitsgerichteten, insbesondere speicherprogrammierbaren, Steuerung ist.

9. Computerprogramm nach einem der Ansprüche 7 bis 8, wobei die Funktionsmodule als Elemente einer Bibliothek für eine fehlersichere Programmierung einer Steuerung bereitgestellt sind.

**Claims**

1. Project engineering system for creating a program for monitoring exothermic reactions in a reactor, wherein, in order to create the program, the project engineering system provides

- at least a first functional module with a mathematical model for ascertaining a maximum temperature and/or a maximum pressure in the reactor in the event of a runaway reaction on the basis of measurement values of physical variables in the reactor and on the basis of substance data of components in the reactor via an ascertaining of concentrations of components in the reactor, and
- at least a second functional module for ascertaining the substance data, in particular a thermal capacity, a density, a vapor pressure, a conductivity, a solubility and/or a viscosity of one or more components in the reactor, wherein at least the second functional module comprises an interface to capture constants of pure substance equations, in particular from a substance database, and
wherein the program comprises commands which, when the program is executed by a computer, prompt it to carry out a method in which, to monitor the exothermic reactions in the reactor, an accumulation of at least one reaction component in the reactor is ascertained, and based on this a maximum temperature and/or a maximum pressure in the reactor in the event of a runaway reaction is ascertained.

2. Project engineering system according to claim 1, wherein the program is a fail-safe program of a safety-related controller, in particular a safety-related programmable logic controller.

3. Project engineering system according to one of claims 1 to 2, wherein the functional modules are provided as elements in a library for fail-safe programming of a controller.

4. Method for creating a program for monitoring exothermic reactions in a reactor, wherein the program is created from

- at least a first functional module with a mathematical model for ascertaining a maximum temperature and/or a maximum pressure in the reactor in the event of a runaway reaction on the basis of measurement values of physical variables in the reactor and on the basis of substance data of components in the reactor via an ascertaining of concentrations of components in the reactor, and
- at least a second functional module for ascertaining the substance data, in particular a thermal capacity, a density, a vapor pressure, a conductivity, a solubility and/or a viscosity of one or more components in the reactor, wherein at least the second functional module comprises an interface to capture constants of pure substance equations, in particular from a substance database, and
wherein the program comprises commands which, when the program is executed by a computer, prompt it to carry out a method in which, to monitor the exothermic reactions in the reactor, an accumulation of at least one reaction component in the reactor is ascertained, and based on this a maximum temperature and/or a maximum pressure in the reactor in the event of a runaway reaction is ascertained.

5. Method according to claim 4, wherein the program is a fail-safe program of a safety-related controller, in particular a safety-related programmable logic controller.

6. Method according to one of claims 4 to 5, wherein the functional modules are provided as elements in a library for fail-safe programming of a controller.

7. Computer program for monitoring exothermic reactions in a reactor, comprising

- at least a first functional module with a mathematical model for ascertaining a maximum temperature and/or a maximum pressure in the reactor in the event of a runaway reaction on the basis of measurement values of physical variables in the reactor and on the basis of substance data of components in the reactor via an ascertaining of concentrations of components in the reactor, and
- at least a second functional module for ascertaining the substance data, in particular a thermal capacity, a density, a vapor pressure, a conductivity, a solubility and/or a viscosity of one or more components in the reactor, wherein at least the second functional module comprises an interface to capture constants of pure substance equations, in particular from a substance database, and
wherein the program comprises commands which, when the program is executed by a computer, prompt it to carry out a method in which, to monitor the exothermic reactions in the reactor, an accumulation of at least one reaction component in the reactor is ascertained, and based on this a maximum temperature and/or a maximum pressure in the reactor in the event of a runaway reaction is ascertained.

8. Computer program according to claim 7, wherein the program is a fail-safe program of a safety-related controller, in particular a safety-related programmable logic controller.

9. Computer program according to one of claims 7 to 8, wherein the functional modules are provided as elements in a library for fail-safe programming of a controller.

**Revendications**

1. Système d'établissement de projet pour l'établissement d'un programme de contrôle de réactions exothermiques dans un réacteur, dans lequel le système d'établissement de projet pour l'établissement du programme dispose

- d'au moins un premier module fonctionnel, ayant un modèle mathématique de détermination d'une température maximum et/ou d'une pression maximum dans le réacteur dans le cas de l'emballement d'une réaction à l'aide de valeurs de mesures de grandeurs physiques dans le réacteur et à l'aide de données de matière de composants dans le réacteur, par l'intermédiaire d'une détermination de concentrations de composants dans le réacteur, et
- d'au moins un deuxième module fonctionnel de détermination des données de matière, en particulier d'une capacité calorifique, d'une masse volumique, d'une tension de vapeur, d'une conductivité, d'une solubilité et/ou d'une viscosité d'un ou de plusieurs composants dans le réacteur,

dans lequel au moins le deuxième module fonctionnel comprend une interface de détection de constantes d'équations de matière pure, en particulier dans une base de données de matière, et

dans lequel le programme comprend des instructions qui, lorsque le programme est exécuté par un ordinateur, font que celui-ci exécute un procédé, dans lequel pour le contrôle des réactions exothermiques dans le réacteur, on détermine une accumulation d'au moins un composant réactionnel dans le réacteur et, sur cette base, on détermine une température maximum et/ou une pression maximum dans le réacteur dans le cas de l'emballement d'une réaction.

2. Système d'établissement de projet suivant la revendication 1, dans lequel le programme est un programme sécurisé de l'erreur d'une commande sécurisée, en particulier programmable par mémoire.

3. Système d'établissement de projet suivant l'une des revendications 1 à 2, dans lequel les modules fonctionnels sont mis à disposition sous la forme d'éléments d'une bibliothèque pour une programmation sécurisée à l'erreur d'une commande.

4. Procédé d'établissement d'un programme de contrôle de réactions exothermiques dans un réacteur, dans lequel on établit le programme à partir

- d'au moins un premier module fonctionnel, ayant un modèle mathématique de détermination d'une température maximum et/ou d'une pression maximum dans le réacteur dans le cas de l'emballement d'une réaction à l'aide de valeurs de mesure de grandeurs physiques dans le réacteur et à l'aide de données de matière de composants dans le réacteur, par l'intermédiaire d'une détermination de concentrations de composants dans le réacteur, et
- d'au moins un deuxième module fonctionnel de détermination des données de matières, en particulier d'une capacité calorifique, d'une masse volumique, d'une tension de vapeur, d'une conductivité, d'une solubilité et/ou d'une viscosité d'un ou de plusieurs composants dans le réacteur,

dans lequel au moins le deuxième module fonctionnel comprend une interface de détection de constantes d'équations de matière pure, en particulier dans une base de données de matière, et

dans lequel le programme comprend des instructions qui, lorsque le programme est exécuté par un ordinateur, font que celui-ci exécute un procédé, dans lequel, pour le contrôle des réactions exothermiques dans le réacteur, on détermine une accumulation d'au moins un composant réactionnel dans le réacteur et, sur cette base, on détermine une température maximum et/ou une pression maximum dans le réacteur dans le cas de l'emballement d'une réaction.

5. Procédé suivant la revendication 4, dans lequel le programme est un programme sécurisé à l'erreur d'une commande de sécurité, en particulier programmable par mémoire.

6. Procédé suivant l'une des revendications 4 à 5, dans lequel les modules fonctionnels sont mis à disposition sous la forme d'éléments d'une bibliothèque pour une programmation sécurisée à l'erreur d'une commande.

7. Programme d'ordinateur pour le contrôle de réactions exothermiques dans un réacteur comprenant

- au moins un premier module fonctionnel, ayant un modèle mathématique de détermination d'une température maximum et/ou d'une pression maximum dans le réacteur dans le cas de l'emballement d'une réaction à l'aide de valeurs de mesure de grandeurs physiques dans le réacteur et à l'aide de données de matière de composants dans le réacteur, par l'intermédiaire d'une détermination de concentrations de composants dans le réacteur, et
- au moins un deuxième module fonctionnel de détermination des données de matières, en particulier d'une capacité calorifique, d'une masse volumique, d'une tension de vapeur, d'une conductivité, d'une solubilité et/ou d'une viscosité d'un ou de plusieurs composants dans le réacteur,

dans lequel au moins le deuxième module fonctionnel comprend une interface de détection de constantes d'équations de matière pure, en particulier dans une base de données de matière, et

dans lequel le programme comprend des instructions qui, lorsque le programme est exécuté par un ordinateur, font que celui-ci exécute un procédé, dans lequel, pour le contrôle des réactions exothermiques dans le réacteur, on détermine une accumulation d'au moins un composant réactionnel dans le réacteur et, sur cette base, on détermine une température maximum et/ou une pression maximum dans le réacteur dans le cas de l'emballement d'une réaction.

8. Programme d'ordinateur suivant la revendication 7, dans lequel le programme est un programme sécurisé à l'erreur d'une commande sécurisée, en particulier programmable par mémoire.

9. Programme d'ordinateur suivant l'une des revendications 7 à 8, dans lequel les modules fonctionnels sont mis à disposition sous la forme d'éléments d'une bibliothèque pour une programmation sécurisée à l'erreur d'une commande.

# FIG 1

# FIG 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0882499 A1
- WO 03103826 A1
- WO 0047632 A1
- EP 3621726 B1
- WO 2021076093 A1
- DE 102018216456 A1

**In der Beschreibung aufgeführte Nicht-Patentliteratur**

- **JOHANNES et al.** Model-based zero emission safety concept for reactors with exothermal reactions for chemical plants.. *Journal of Loss Prevention in the Process Industries*, 2021, vol. 72, 104494
- **SCHMIDT C. ; BIERNATH J. ; SCHMIDT J. ; DENECKE J.** Protection of chemical reactors against exothermal runaway reactions with smart overpressure protection devices. *Chemical Engineering Transactions*, 2022, vol. 90, 493-498
- **BATES R. et al.** Implementation of Ultrasonic Sensing for High Resolution Measurement of Binary Gas Mixture Fractions. *Sensors*, 2014, vol. 14, 11260-11276